# EUROPÄISCHE PATENTSCHRIFT

(11) **EP 3 950 914 B1**
(45) Veröffentlichungstag und Bekanntmachung des Hinweises auf die Patenterteilung: **07.06.2023**
(21) Anmeldenummer: 20189166.0
(22) Anmeldetag: 03.08.2020
(51) Int. Cl.: C12F 3/10, C12P 7/06, C12P 5/02

(54) **VERFAHREN ZUR DURCHFÜHRUNG EINES KOMBINIERTEN BETRIEBS EINER BIOETHANOLGEWINNUNGSANLAGE UND EINER BIOGASANLAGE**
METHOD FOR A COMBINED OPERATION OF A BIOETHYLENE RECOVERY PLANT AND A BIOGAS PLANT
PROCÉDÉ DE MISE EN UVRE D'UN FONCTIONNEMENT COMBINÉ D'UNE INSTALLATION DE PRODUCTION DE BIOÉTHANOL ET D'UNE INSTALLATION DE BIOGAZ

(43) Veröffentlichungstag der Anmeldung: 09.02.2022
(73) Patentinhaber: Verbio Vereinigte Bioenergie AG, 04109 Leipzig (DE)
(72) Erfinder: Bonk, Fabian, 04315 Leipzig (DE); Schlimbach, Michael, 06120 Halle/Saale (DE); Lüdtke, Oliver, 04416 Markkleeberg (DE)
(74) Vertreter: Gulde & Partner

(56) Entgegenhaltungen:
- WO-A1-2017/076380
- DE-A1-102013 226 991
- DE-A1-102014 001 912
- US-A1- 2007 141 691

## Beschreibung

Die Erfindung betrifft ein Verfahren zur Durchführung eines kombinierten Betriebs einer Bioethanolgewinnungsanlage und einer Biogasanlage.

### Technologischer Hintergrund

Die Produktion von Ethanol aus Mais ist bekannt und insbesondere in den USA eine großtechnisch breit umgesetzte Technologie. Stand der Technik sind Anlagen mit folgenden Prozessschritten:
- Trockenvermahlung von Mais mit Siebgröße 2-4 mm (Jacques, Lyons, & Kelsall, 2003), wobei ein Massenanteil von ca. 33 % der Partikel des dabei entstehenden Maismehls eine Korngröße <0,59 mm aufweisen (Jacques, Lyons, & Kelsall, 2003),
- Anmaischung von Maismehl mit Prozessflüssigkeiten, dass sich vor allem aus Dünnschlempe (10-50 % des Prozessflüssigkeiten (Jacques, Lyons, & Kelsall, 2003)) und Kondensat der Futtermitteleindampfung zusammensetzt. Der Trockensubstanzgehalt (TS-Gehalt) der Maische beträgt ca. 29-33 % (Jacques, Lyons, & Kelsall, 2003). Der TS-Gehalt der Dünnschlempe beträgt ca. 4,4-7,8 % (Jacques, Lyons, & Kelsall, 2003). Damit werden ca. 0,01-0,08 t TS in der Form von Dünnschlempe pro Tonne Maismehl für die Anmaischung eingesetzt.
- In einem typischen Fermentationsprozess werden ca. 360 mg Stickstoff pro L Maische zum Wachstum der Hefen benötigt (Jacques, Lyons, & Kelsall, 2003). Fehlender Stickstoff wird typischerweise als Harnstoff dem Prozess zugeführt.
- Die Maische wird einer Kochstufe zugeführt. Hier wird die Maische typischerweise auf ca. 104-107 °C (Walker, Abbas, Ingledew, & Pilgrim, 2017) erhitzt, dann die Temperatur in einem Flash-Behälter auf ca. 85 °C reduziert und unter Zugabe von Enzymen die Stärke aufgeschlossen. Damit wird eine Maischetemperatur weit über der Verkleisterungstemperatur von Mais eingestellt, die bei 62-72 °C für Standardmais bzw. 67-80 °C für Mais mit hohem Amyloseanteil liegt (Walker, Abbas, Ingledew, & Pilgrim, 2017).
- Die Maische der Kochstufe wird einer Fermentation zugeführt und die aufgeschlossene Stärke zwischen 25-35 °C (Walker, Abbas, Ingledew, & Pilgrim, 2017) von Hefen zu Ethanol umgesetzt. Der Massenanteil an nicht umgesetzter Stärke, der Reststärkegehalt, beträgt weniger als 1,5 %.
- Etwa 4% der im Maismehl vorhandenen Zucker und Stärke (Medina et al., 2009) werden statt zu Ethanol zum unerwünschten Nebenprodukt Glycerin umgewandelt, was ca. 24 kg Glycerin pro Tonne Maismehl entspricht.
- Die ethanolhaltige Maische aus der Fermentation wird einer Destillation bei ca. 108 °C zugeführt (Walker, Abbas, Ingledew, & Pilgrim, 2017) und Ethanol aus der Maische entfernt. Die dabei entstehende ethanolarme Maische wird als Dickschlempe bezeichnet.
- Die gesamte Dickschlempe wird einer Fest-Flüssigtrennung, meistens Dekantierzentrifugen, zugeführt. Der TS-Gehalt der Festphase, nachfolgend als Schlempefeststoff bezeichnet, beträgt ca. 29-39 % (Walker, Abbas, Ingledew, & Pilgrim, 2017) und der TS-Gehalt der Flüssigphase, nachfolgend als Dünnschlempe bezeichnet, ca. 4,4-7,8 % (Jacques, Lyons, & Kelsall, 2003).
- Ein Teil der Dünnschlempe wird zum Anmaischen des Maismehls verwendet. Der Rest wird zum sogenannten Syrup eingedampft und das Kondensat als Prozessflüssigkeit in der Anmaischung eingesetzt. Aus dem Syrup kann Maisöl abgetrennt und gesondert verwertet werden.
- Syrup und Schlempefeststoff werden als Futtermittel verwendet. Syrup und Schlempefeststoff werden häufig getrocknet, können aber auch feucht als Futtermittel eingesetzt werden. Insgesamt werden typischerweise ca. 0,25 t Trockensubstanz an Futtermittel pro Tonne Maismehl produziert.

Dieser Standardprozess weist folgende Nachteile auf:
- Kochstufe und Destillation werden bei hohen Temperaturen betrieben, was zu einem hohen Wärmeenergieeinsatz führt.
- Die Fest-Flüssigtrennung der kompletten Menge an anfallender Dickschlempe führt zu einen hohen elektrischen Energieeinsatz.
- Die Dünnschlempe wird eingedampft, um Prozessflüssigkeiten zu gewinnen, was zu einem hohen Wärmeenergieeinsatz führt.
- Typische weitere Trocknungsprozesse von Syrup und Schlempefeststoff bewirken einen hohen Wärmeenergieeinsatz, sind in der typischen Anlagenkonfiguration aber notwendig, um ein wirtschaftlich attraktiveres Futtermittel zu gewinnen.
- Stickstoff muss in der Form von Harnstoff oder ähnlichen Chemikalien zugegeben werden.
- Die genannten Energie- und Chemikalienverbräuche wirken sich negativ auf die Treibhausgasbilanz der produzierten Kraftstoffe aus. Eine Quantifizierung der negativen Auswirkungen bietet zum Beispiel der carbon intensity score (CI score) nach dem California Low-Carbon Fuel Standard. Der CI score ist ein Maß für die Treibhausgasemissionen von Kraftstoffen in Gramm Kohlenstoffdioxid-Äquivalenten (gCO₂e). Eine typische Ethanolanlage in den USA produziert Kraftstoffe mit einem CI score von ca. 79 gCO2e/MJ (https://ww3.arb.ca.gov/fuels/lcfs/lcfs_meetings/01312017discussionpaper_etoh.p df (Appendix A, Abgerufen am 17.06.2020). Der Herstellungsprozess in der Ethanolanlage macht davon ca. 32 gCO₂e/MJ¹ aus, der Rest sind andere Emissionen in der Prozesskette wie z.B. der Maisanbau.
- Eine typische Ethanolanlage weist einen hohen Wasserverbrauch von ca. 2,7 L Frischwasser pro L Ethanol auf (Mueller, 2010), was ca. 1,2 m³ pro Tonne Maismehl entspricht.

Kochstufen bei Temperaturen unter der Verkleisterungstemperatur von Stärke, sogenannte Kaltmaischverfahren, sind bekannt und bewirken einen niedrigeren Wärmeenergieeinsatz, führen aber zu hohen Verlusten durch Reststärke und der Gefahr von bakterieller Kontamination. Die Verkleinerung der Korngröße des Maismehls, die Änderung des pH-Werts und ein höherer Enzymeinsatz können diese Verluste und Nachteile verringern (Walker, Abbas, Ingledew, & Pilgrim, 2017). Diese bekannten Maßnahmen allein haben aber in der Vergangenheit nicht ausgereicht, damit sich das Kaltmaischverfahren gegen das Heißmaischverfahren durchsetzen konnte.

Des Weiteren sind Verfahren bekannt, die den Energieeinsatz der Futtermittelherstellung einsparen, in dem Schlempe zu Biogas statt zu Futtermittel verarbeitet wird. EP2501818B1 beschreibt ein Verfahren, bei dem die Schlempe einer Ethanolanlage einer Biogasanlage zugeführt wird und ein Teil des Ablaufs der Biogasanlage direkt in die Ethanolanlage zurückgeführt wird. US8962309B2 beschreibt eine Biogasanlage mit verschiedenen Typen an Biogasfermentern für Dünnschlempe und Schlempefeststoff. Die Biogasfermenter sollen dabei bei einer Ammoniumkonzentration von unter 6000 ppm NH4-N betrieben werden. WO2013000925A1 beschreibt ein Verfahren, in dem Dünnschlempe einem Biogasfermenter des Typs Rührkesselreaktor (CSTR) bei einer durchschnittlichen hydraulischen Verweilzeit von 1-20 Tagen zugeführt wird.

US 2007/0141691 A1 offenbart ein Verfahren zur Herstellung von Ethanol und Energie. Das Verfahren umfasst die Schritte der Fermentierung einer Maismaische in einem wässrigen Medium zur Herstellung eines Biers. Anschließend wird das Bier destilliert, wobei Ethanol und Schlempe anfallen. Die gesamte Schlempe wird anaerob vergoren, um ein Biogas und einen Rückstand zu erzeugen.

DE 10 2013 226 991 A1 betrifft ein Verfahren zur Entfernung von organischen Säuren und/oder aromatischen Verbindungen in wässrigen Medien zum Zweck der Aufbereitung, Rückgewinnung und/oder Wiederverwertung als Prozesswasser.

Der im Patentanspruch 1 angegebenen Erfindung liegt das Problem zugrunde, eine, im Vergleich zur oben beschriebenen typischen Ethanolanlage, energie-, wasser- und chemikalieneinsparende Kombination aus Bioethanolanlage und Biogasanlage zu betreiben, die trotz Energieeinsparungen hohe Ethanolausbeuten pro Tonne Maismehl aufweist. Auch sollte sich der Einsatz von Ablauf aus der Biogasanlage als Prozessflüssigkeiten in der Ethanolanlage nicht negativ auf die Ethanolfermentation auswirken.

### Zusammenfassung der Erfindung

Die vorliegende Erfindung greift die zuvor beschriebene Aufgabenstellung auf und löst diese durch Bereitstellen eines Verfahrens zur Durchführung eines kombinierten Betriebs einer Bioethanolgewinnungsanlage und einer Biogasanlage mit den in Anspruch 1 genannten Schritten:
a) Maismehl aus einer Trockenvermahlung wird mindestens mit 0,1 t Trockensubstanz in Form von Dickschlempe und mindestens 0,1 m³ Ablauf der Biogasanlage pro Tonne Maismehl angemaischt,
b) Die Maische aus a) wird einer Kochstufe mit Maischetemperaturen unterhalb der Verkleisterungstemperatur der Stärke im Maismehl, nachfolgend einer ethanolbildenden Fermentation und die fermentierte Maische nachfolgend einer Destillation zugeführt,
c) Dickschlempe aus b) wird der Anmaischung in a) und der Biogasanlage zugeführt.

Im Schritt a) wird Maismehl aus einer Trockenvermahlung von Mais mit Flüssigkeiten angemaischt. Diese Flüssigkeiten bestehen pro Tonne Maismehl zumindest aus 0,1 t TS Dickschlempe und zumindest aus 0,1 m³ Ablauf aus der Biogasanlage.

Überraschend wurde festgestellt, dass durch hohe Rückführung von Trockensubstanz in Form von Dickschlempe die Vorteile der Energieeinsparungen von Kochstufe und Maischedestillation bei niedrigen Temperaturen realisiert werden können und gleichzeitig niedrige Reststärkegehalte in der fermentierten Maische, und damit geringere Ethanolausbeuteverluste möglich sind. Des Weiteren wird durch die hohe Rückführung der Massenanteil von Stärke und Zucker, der zum ungewollten Nebenprodukt Glycerin umgewandelt wird, von typischerweise 4% auf unter 2,5% reduziert. Auch erhöht sich durch die hohe Rückführung von Dickschlempe die Verfügbarkeit von Stickstoff für das Hefewachstum. Dadurch können hohe Ethanolausbeuten von über 435 Litern pro Tonne Maismehl erreicht werden.

Die Rückführung von Ablauf aus der Biogasanlage hat den Vorteil, dass Frischwasser sowie Prozessflüssigkeiten eingespart werden. In einer typischen Ethanolanlage werden Prozessflüssigkeiten energieintensiv über die Eindampfung von Dünnschlempe gewonnen.

In Schritt b) wird die Maische aus Schritt a) einer Kochstufe zugeführt, in der die Maische auf Temperaturen unterhalb der Verkleisterungstemperatur der Stärke im Maismehl aufgeheizt wird. Dies führt zu deutlichen Energieeinsparungen im Vergleich zu typischen Kochstufen bei 104-107°C. Die Maische aus der Kochstufe wird einer Fermentation zugeführt, in der Ethanol gebildet wird. Die ethanolhaltige Maische aus der Fermentation wird dann einer Destillation zugeführt, bei der Ethanol abgetrennt wird.

In Schritt c) wird ein Teil der ethanolarmen Maische aus der Destillation, der sogenannte Dickschlempe, ohne die typische Fest-Flüssigtrennung direkt der Anmaischung in Schritt a) und der Biogasanlage zugeführt. Im Vergleich zur typischen Fest-Flüssigtrennung der gesamten Dickschlempe wird dadurch elektrische Energie für die Fest-Flüssigtrennung von Dickschlempe eingespart und ein großer Teil der Reststärke aus der Fermentation zurückgeführt, der sonst als Futtermittel nicht mehr der Ethanolbildung zur Verfügung stehen würde.

### Detaillierte Beschreibung der Erfindung

### Definitionen

Im Zusammenhang der vorliegenden Erfindung wird unter der Trockensubstanz (TS) der Feststoffrückstand verstanden, welcher nach Entfernen des Lösungsmittels (z.B. Wasser oder Ethanol) aus einer Suspension (z.B. aus einer Schlempe) oder aus einer Lösung erhalten wird. Das heißt, der Feststoffrückstand ist als Gesamtheit aller zuvor gelösten oder suspendierten Feststoffe (z.B. Rohproteine, Hefe und Salze) zu verstehen. Die Masse der Trockensubstanz wird Trockenmasse genannt und kann in Kilogramm angegeben werden. Unter Trockensubstanzgehalt (TS-Gehalt) wird der prozentuale Massenanteil der Trockensubstanz bezogen auf die Gesamtmasse der Suspension (z.B. der Schlempe) oder Lösung verstanden.

Im Zusammenhang der vorliegenden Erfindung wird unter dem Begriff "Schlempe" der Rückstand aus der Destillation einer ethanolhaltigen Getreidemaische verstanden. Der Begriff "Dickschlempe" wird synonym für Schlempe verwendet.

Im Zusammenhang der vorliegenden Erfindung wird unter "Fest-Flüssig-Trennung" ein Verfahren verstanden, das eine Suspension (z.B. Schlempe) in ein Zweiphasen-System, umfassend eine Fest-Phase und eine Flüssig-Phase, separiert. Die Fest-Flüssig-Trennung kann vorzugsweise in einem Separator oder Dekanter erfolgen. Unter einer Fest-Phase wird in einem Zweiphasen-System die Phase verstanden, die den höheren Trockensubstanzgehalt aufweist. Eine Fest-Phase kann dabei eine Suspension oder einen sedimentierten Feststoff (Rückstand) umfassen. Unter einer Flüssig-Phase wird in einem Zweiphasen-System die Phase verstanden, die den geringeren Trockensubstanzgehalt aufweist. Eine Flüssig-Phase kann dabei eine Suspension oder klare Lösung umfassen.

Im Zusammenhang der vorliegenden Erfindung wird unter "Dünnschlempe" eine durch eine Fest-Flüssig-Trennung von Dickschlempe erzeugte flüssige Phase verstanden. Der TS-Gehalt einer Dünnschlempe kann vorzugsweise mindestens 8 % betragen. Der Begriff "Schlempefeststoff" steht innerhalb dieser Erfindung für die feste Phase, die durch eine Fest-Flüssig-Trennung aus der Schlempe abgetrennt wird.

Im Zusammenhang der vorliegenden Erfindung wird unter "Ablauffeststoff" die feste Phase verstanden, die durch eine Fest-Flüssig-Trennung aus dem Ablauf eines Biogasfermenters abgetrennt wird.

Im Zusammenhang der vorliegenden Erfindung wird unter "Ammoniumstickstoffgehalt" (NH4-N Gehalt) der Massenanteil an Stickstoff in Form von Ammonium in einer Probe verstanden. Zur Messung des NH4-N Gehalts in einer Probe, wird die Probe mit Natronlauge basisch gestellt und anschließend mit Wasserdampf destilliert. Ammoniak wird aus der Probe ausgetrieben und in einer Borsäurevorlage aufgefangen. Die Bestimmung des Massenanteils an Ammoniumstickstoff erfolgt durch Titration des entstandenen Borats mit verdünnter Salzsäure.

### Beschreibung der Erfindung

Mais wird einer Trockenvermahlung zugeführt, um die Partikelgröße zu reduzieren. In einer bevorzugten Ausführungsform kann das erfindungsgemäße Verfahren so ausgestaltet sein, dass der Massenanteil der Partikel mit einer Korngröße <0,5 mm nach der Vermahlung mindestens 60 %, bevorzugt mindestens 65 %, besonders bevorzugt mindestens 70 % des Maismehls beträgt. In einer weiteren bevorzugten Ausführungsform kann das erfindungsgemäße Verfahren so ausgestaltet sein, dass der Massenanteil der Partikel mit einer Korngröße <0,36 mm nach der Vermahlung mindestens 45 %, bevorzugt mindestens 50 %, besonders bevorzugt mindestens 55 % beträgt. Diese deutlich kleineren Korngrößen im Vergleich zum Stand der Technik haben den Vorteil, dass die Kornoberfläche besser für stärkeabbauende Enzyme zugänglich ist. Des Weiteren wurde überraschend festgestellt, dass so das Absetzen von Partikeln in Fermentern deutlich reduziert werden kann, was beim Einsatz eines Kaltmaischverfahrens sonst zu hohen Reststärkegehalten in der fermentierten Maische und zu Produktivitätseinbüßen führt.

Das Maismehl aus der Trockenvermahlung wird mit verschiedenen Flüssigkeiten angemaischt. Diese Flüssigkeiten sind zumindest Dickschlempe und Ablauf aus der Biogasanlage. Zusätzlich können auch andere Flüssigkeiten wie z.B. Dünnschlempe oder Prozessflüssigkeiten eingesetzt werden.

Überraschend wurde festgestellt, dass durch eine hohe Rückführung von Trockensubstanz in Form von Dickschlempe in die Anmaischung die Vorteile der Energieeinsparungen von Kochstufe und Maischedestillation bei niedrigen Temperaturen realisiert werden können und gleichzeitig niedrige Ethanolausbeuteverluste durch Reststärke möglich sind. In einer bevorzugten Ausführungsform ist das Verfahren so ausgestaltet, dass pro Tonne Maismehl in der Destillation mindestens 400 Liter Ethanol, bevorzugt mindestens 425 Liter Ethanol, besonders bevorzugt mindestens 435 Liter Ethanol abgetrennt werden. Dazu müssen mindestens 0,1 t TS in Form von Dickschlempe pro Tonne Maismehl in die Anmaischung zurückgeführt werden. In einer bevorzugten Ausführungsform kann das erfindungsgemäße Verfahren so ausgestaltet sein, dass mindestens 0,12 t TS, bevorzugt mindestens 0,14 t TS, besonders bevorzugt mindestens 0,16 t TS in Form von Dickschlempe pro Tonne Maismehl in die Anmaischung zurückgeführt werden.

In einer weiteren bevorzugten Ausführungsform kann das erfindungsgemäße Verfahren so ausgestaltet sein, dass mindestens 0,2 t TS, bevorzugt mindestens 0,3 t TS, besonders bevorzugt mindestens 0,4 t TS in Form eines Gemischs aus Dünn- und Dickschlempe pro Tonne Maismehl zugegeben werden. In einer bevorzugten Ausführungsform kann das erfindungsgemäße Verfahren so ausgestaltet sein, dass das Gemisch aus Dünn- und Dickschlempe einen Volumenanteil von mindestens 10 %, bevorzugt mindestens 20 %, besonders bevorzugt mindestens 30 % an Dickschlempe enthält.

In einer bevorzugten Ausführungsform werden mindestens 0,1 t TS, bevorzugt mindestens 0,2 t TS, bevorzugt mindestens 0,25 t TS Dünnschlempe pro t Maische zurückgeführt. Die Rückführung größerer Mengen an Dünnschlempe ist von Vorteil im Vergleich zum Einsatz von Frischwasser oder von Prozessflüssigkeiten, die energieintensiv über die Eindampfung von Dünnschlempe gewonnen werden.

In einer bevorzugten Ausführungsform ist das Verfahren so ausgestaltet, dass pro Tonne Maismehl mindestens 15 kg, bevorzugt mindestens 18 kg, besonders bevorzugt mindestens 20 kg Glycerin über Dick- und Dünnschlempe in die Anmaischung zurückgefahren wird. Überraschenderweise wurde festgestellt, dass eine hohe Rückführung von Glycerin die Neubildung von Glycerin in der Ethanolfermentation signifikant reduziert. Dadurch geht weniger Stärke oder Zucker als Glycerin verloren und damit wird die Ethanolausbeute gesteigert. In einer bevorzugten Ausführungsform ist das Verfahren so ausgestaltet, dass die Rückführung des Gemisches aus Dünn- und Dickschlempe so hoch gewählt wird, dass ein Massenanteil von weniger als 2,5 %, bevorzugt weniger als 2,3 %, besonders bevorzugt weniger als 2,1 % der im Maismehl vorhandenen Stärke und Zucker in Glycerin umgewandelt werden. In einer bevorzugten Ausführungsform ist das Verfahren so ausgestaltet, dass die Rückführung des Gemisches aus Dünn- und Dickschlempe so hoch gewählt wird, dass weniger als 19 kg, bevorzugt weniger als 17 kg, besonders bevorzugt weniger als 15 kg Glycerin pro Tonne Maismehl produziert werden.

Der Anteil an Ablauf aus der Biogasanlage beträgt mindestens 0,1 m³ pro Tonne Maismehl. Die Rückführung von Ablauf aus der Biogasanlage hat den Vorteil, dass Frischwasser sowie Prozessflüssigkeiten eingespart werden. Prozessflüssigkeiten werden typischerweise energieintensiv über die Eindampfung von Dünnschlempe gewonnen. Überraschend wurde festgestellt, dass die Zugabe von Ablauf aus der Biogasanlage zur Anmaischung nicht zu einer starken Inhibierung der Enzyme und Hefen im Ethanolprozess führt, wenn die Zufuhr von NH4-N in Form von Ablauf aus der Biogasanlage begrenzt wird. In einer bevorzugten Ausführungsform ist das Verfahren so ausgestaltet, dass in der Anmaischung maximal 1000 g, bevorzugt maximal 800 g, besonders bevorzugt maximal 600 g NH4-N pro Tonne Maismehl über den Ablauf der Biogasanlage zurückgeführt werden. Andererseits sollte immer eine gewisse Menge an NH4-N Rückführung in die Anmaischung angestrebt werden, um den Einsatz von externen Stickstoffquellen wie Harnstoff zu reduzieren bzw. obsolet zu machen. In einer bevorzugten Ausführungsform ist das Verfahren so ausgestaltet, dass in der Anmaischung mindestens 100 g, bevorzugt mindestens 200 g, besonders bevorzugt mindestens 400 g Ammoniumstickstoff pro Tonne Maismehl über den Ablauf der Biogasanlage zurückgeführt werden. In einer bevorzugten Ausführungsform beträgt der Anteil an Ablauf aus der Biogasanlage pro Tonne Maismehl mindestens 0,2 m³, bevorzugt mindestens 0,4 m³, besonders bevorzugt mindestens 0,8 m³.

In einer bevorzugten Ausführungsform kann das erfindungsgemäße Verfahren so ausgestaltet sein, dass über den Ablauf aus der Biogasanlage auch Cellulasen und cellulasebildenden Mikroorganismen aus der Biogasanlage der Anmaischung zugeführt werden. Dies hat den Vorteil, dass damit den Hefen in der Fermentation auch Glucose aus der enzymatischen Hydrolyse von Cellulose zur Verfügung stehen und damit die Ethanolausbeute erhöht werden kann. Mit der erfindungsgemäßen hohen Rückführung an Dickschlempe werden im Vergleich zu einer typischen Ethanolanlage auch große Mengen an Cellulose in die Anmaischung zurückgefahren und stehen so der enzymatischen Hydrolyse zur Verfügung. Cellulose macht vorzugsweise einen Massenanteil von mindestens 5 % der TS von Dickschlempe aus. Über die Rückführung von mindestens 0,1 t TS in die Anmaischung werden so mindestens 5 kg Cellulose zurückgeführt. Der Cellulosegehalt der Dickschlempe wird über eine Futtermittelanalyse nach VDLUFA III bestimmt. Der Cellulosegehalt wird berechnet als Messwert ADF ("acid detergent fiber") abzüglich dem Messwert ADL ("acid detergent lignin") einer Dickschlempeprobe.

In einer weiteren Ausführungsform kann das erfindungsgemäße Verfahren so ausgestaltet sein, dass in der Anmaischung Prozessflüssigkeiten zusätzlich zu Dickschlempe und Ablauf aus der Biogasanlage eingesetzt werden. Prozessflüssigkeiten können hierbei ausgewählt sein aus der Gruppe umfassend: Dünnschlempe, Lutterwasser, Rohwasser, Trinkwasser, Nutzwasser, Regenwasser, Grundwasser, Oberflächenwasser, Kondensate aus einer Eindampfung von Dünnschlempe, Prozesswasser aus CO₂-Wäschern, Absalzwasser aus Kühltürmen, Absalzwasser und Abschlämmwasser aus Kesseln zur Dampferzeugung, und Mischungen dergleichen. In einer bevorzugten Ausführungsform kann das erfindungsgemäße Verfahren so ausgestaltet sein, dass pro Tonne Maismehl weniger als 1,2 m³, bevorzugt weniger als 0,8 m³, besonders bevorzugt weniger als 0,2 m³ Frischwasser eingesetzt werden.

In einer bevorzugten Ausführungsform kann das erfindungsgemäße Verfahren so ausgestaltet sein, dass der pH-Wert der Maische auf kleiner 4,5, bevorzugt auf kleiner 4,2, besonders bevorzugt auf kleiner 3,8 eingestellt wird. Überraschenderweise wurde festgestellt, dass sich so trotz Kaltmaischverfahren eine Kontamination durch Bakterien in der Ethanolfermentation vermeiden lässt, sich Proteine besser aufkonzentrieren lassen, sowie sich die Verfügbarkeit der Cellulose im Maismehl zum biologischen Abbau in der Ethanol- und Biogasanlage erhöhen lässt.

Die Maische wird einem Kaltmaischverfahren unterzogen, das heißt einer Kochstufe zugeführt, in der die Maische auf Temperaturen unterhalb der Verkleisterungstemperatur der Stärke im Maismehl erwärmt wird. Dies führt zu deutlichen Energieeinsparungen im Vergleich zu typischen Kochstufen bei 104-107 °C. In einer bevorzugten Ausführungsform kann das erfindungsgemäße Verfahren so ausgestaltet sein, dass die Maische auf maximal 70 °C, bevorzugt auf maximal 66 °C, besonders bevorzugt auf maximal 64 °C erwärmt wird.

Die Maische aus der Kochstufe wird einer Fermentation zugeführt, in der Ethanol gebildet wird. Die ethanolhaltige Maische aus der Fermentation wird dann einer Destillation zugeführt, bei der Ethanol abgetrennt wird. Dies führt zu deutlichen Energieeinsparungen im Vergleich zu typischen Maischedestillation bei ca. 108 °C. In einer bevorzugten Ausführungsform kann das erfindungsgemäße Verfahren so ausgestaltet sein, dass die ethanolhaltige Maische auf maximal 87°C, bevorzugt auf maximal 79 °C, besonders bevorzugt auf maximal 68 °C erwärmt wird. Die niedrigen Temperaturen der Kochstufe und Destillation führen neben der Energieeinsparung auch dazu, dass Proteine in der Dickschlempe in einer höheren Qualität vorliegen. Dies kann genutzt werden, um hochwertige Futtermittel und Lebensmittel aus der Dick- bzw. Dünnschlempe zu gewinnen.

Die ethanolarme Maische aus der Destillation, die sogenannte Dickschlempe, wird mehreren Verwertungswegen zugeführt. Ein Teil wird direkt in die Anmaischung zurückgeführt. Ein weiterer Teil wird direkt der Biogasanlage zugeführt. Der direkte Einsatz von Dickschlempe hat folgende Vorteile gegenüber der typischen Fest-Flüssigtrennung der gesamten Dickschlempe: Einmal wird elektrische Energie für die Fest-Flüssigtrennung eingespart. Zum anderen wird ein großer Teil der Reststärke aus der Fermentation zurückgeführt, der sonst als Futtermittel oder Substrat für die Biogasanlage nicht mehr der Ethanolbildung zur Verfügung stehen würde.

In einer weiteren Ausführungsform kann das erfindungsgemäße Verfahren so ausgestaltet sein, dass ein weiterer Teil der Dickschlempe einer Fest-Flüssigtrennung zugeführt wird, um Dünnschlempe zu erzeugen. In einer bevorzugten Ausführungsform kann das erfindungsgemäße Verfahren so ausgestaltet sein, dass ein Volumenanteil von maximal 70 %, bevorzugt von maximal 50 %, besonders bevorzugt von maximal 30 % der Dickschlempe einer Fest-Flüssig-Trennung zugeführt wird, um Dünnschlempe und Schlempefeststoff zu generieren. Die Dünnschlempe kann z.B. in der Anmaischung, aber auch zur Gewinnung von Maisöl, Futtermittel oder Lebensmitteln eingesetzt werden. In einer weiteren Ausführungsform kann das erfindungsgemäße Verfahren so ausgestaltet sein, dass ein weiterer Teil der Dickschlempe als Futtermittel verwendet wird. Der Schlempefeststoff kann auch als Futtermittel verwendet werden. In einer weiteren bevorzugten Ausführungsform werden pro Tonne Maismehl maximal 0,18 t TS, bevorzugt maximal 0,12 t TS, besonders bevorzugt maximal 0,06 t TS Futtermittel ausgewählt aus der Gruppe umfassend Dickschlempe, Schlempefeststoff, Syrup (Dünnschlempekonzentrat) und deren getrocknete Formen produziert. In einer bevorzugten Ausführungsform kann das erfindungsgemäße Verfahren so ausgestaltet sein, dass der Schlempefeststoff der Biogasanlage zugeführt wird.

In einer weiteren Ausführungsform kann das erfindungsgemäße Verfahren so ausgestaltet sein, dass ein Proteinprodukt mit einem Rohproteingehalt (Massenanteil Rohprotein an TS Proteinprodukt) größer 44% aus Dünnschlempe gewonnen wird. In einer weiteren Ausführungsform kann das erfindungsgemäße Verfahren so ausgestaltet sein, dass ein Proteinprodukt mit einem Rohproteingehalt (Massenanteil Rohprotein an TS Proteinprodukt) größer 70% aus Dünnschlempe gewonnen wird. In einer weiteren Ausführungsform kann das erfindungsgemäße Verfahren so ausgestaltet sein, dass Maisöl aus Dünnschlempe gewonnen wird. In einer bevorzugten Ausführungsform werden die Reststoffe aus der Gewinnung von Proteinprodukten und Reststoffe aus der Gewinnung von Maisöl der Biogasanlage zugeführt.

Die Biogasanlage besteht mindestens aus einem Biogasfermenter des Typs Rührkesselreaktor (CSTR), in dem Bestandteile des zugeführten Substrats durch eine Mischkultur von Bakterien und Archaeen zu Biogas und Nebenprodukten wie Ammonium umgewandelt werden. Das zugeführte Substrat beinhaltet zumindest Dickschlempe und kann in einer bevorzugten Ausführungsform auch Schlempefeststoff sowie Reststoffe aus der Gewinnung von Maisöl und der Gewinnung von Proteinprodukten beinhalten.

Abhängig von den gewählten Prozessparametern können bei der anaeroben Vergärung bestimmte ungewollte Metabolite akkumulieren. Das sind unter anderem organische Säuren wie z.B. Essig-, Propion-, Butter-, Isobutter-, Valerian-, Isovalerian-, und Capronsäuren, und aromatische Komponenten wie z.B. Phenol, Indol, Skatol oder Kresole. Diese können die Ethanolfermentation in der Ethanolanlage negativ beeinträchtigen und zu Geruchsbelästigung führen.

Hohe Ammoniumkonzentrationen können die anaerobe Vergärung hemmen.

Typischerweise wird daher der NH4-N Gehalt, z.B. durch Verdünnung mit Prozessflüssigkeiten, auf unter 6000 ppm kontrolliert. Allerdings senkt eine Verdünnung die durchschnittliche Verweilzeit der zu vergärenden Dickschlempe im Biogasfermenter, was zu einem ungewollten Rückgang der Biogasausbeute oder einer Akkumulation von Metaboliten führen kann. Überraschend wurde festgestellt, dass eine stabile und effiziente anaerobe Vergärung von Dickschlempe auch bei höheren Ammoniumkonzentrationen möglich ist. Das hat den Vorteil, dass übermäßige Verdünnungen und damit eingehende Verringerung der Verweilzeit, Energieverbräuche und Wasserverbräuche reduziert werden. In einer bevorzugten Ausführungsform kann das erfindungsgemäße Verfahren so ausgestaltet sein, dass die Ammoniumkonzentration in den Biogasfermentern zwischen 6000-9000 ppm, bevorzugt zwischen 7000-9000 ppm, besonders bevorzugt zwischen 7500-9000 ppm geregelt wird. In einer weiteren Ausführungsform kann das erfindungsgemäße Verfahren so ausgestaltet sein, dass die Temperatur in den Biogasfermentern der Biogasanlage auf unter 43°C geregelt wird. Dies ist vorteilhaft, da sich bei höheren Temperaturen das chemische Gleichgewicht von Ammonium zu Ammoniak verschiebt und Ammoniak eine stärkere inhibierende Wirkung auf die anaerobe Vergärung hat.

Überraschend wurde festgestellt, dass selbst bei hohen Ammoniumkonzentrationen eine stabile Vergärung sowie niedrige Gehalte an ungewollten Metaboliten durch eine Kaskadenschaltung von Biogasfermentern erreicht werden kann. In einer bevorzugten Ausführungsform kann das erfindungsgemäße Verfahren so ausgestaltet sein, dass mindestens drei, bevorzugt mindestens vier Biogasfermenter in der Biogasanlage in einer Kaskade betrieben werden. Die Fütterung erfolgt in die ersten beiden Stufen der Kaskade bzw. bevorzugt nur in die erste Stufe der Kaskade.

Um eine stabile Vergärung sowie niedrige Gehalte an ungewollten Metaboliten auch bei hohen Ammoniumkonzentrationen zu gewährleisten, ist eine ausreichend lange durchschnittliche hydraulische Verweilzeit in der Biogasanlage entscheidend. In einer bevorzugten Ausführungsform kann das erfindungsgemäße Verfahren so ausgestaltet sein, dass die durchschnittliche hydraulische Verweilzeit der Biogasanlage mindestens 30 Tage, bevorzugt mindestens 50 Tage, besonders bevorzugt mindestens 70 Tage beträgt. In einer bevorzugten Ausführungsform kann das Verfahren so ausgestaltet sein, dass die hydraulische Verweilzeit so hoch gewählt wird, dass die Konzentrationen von organischen Säuren und aromatischen Verbindungen im Ablauf der Biogasanlage maximal je 450 ppm, bevorzugt maximal je 300 ppm, besonders bevorzugt maximal je 150 ppm beträgt.

In einer weiteren Ausführungsform kann das erfindungsgemäße Verfahren so ausgestaltet sein, dass der Ablauf der Biogasfermenter einer Fest-Flüssigtrennung unterzogen wird, und die feste Phase, der Ablauffeststoff, ausgeschleust wird. In einer bevorzugten Ausführungsform wird der Ablauffeststoff zur Düngung und Bodenverbesserung verwendet.

In einer bevorzugten Ausführungsform kann das erfindungsgemäße Verfahren so ausgestaltet sein, dass der Ablauf der Biogasfermenter bzw. bevorzugt die flüssige Phase einer Fest-Flüssigtrennung des Ablaufs der Biogasfermenter einer Ammoniakstrippung zugeführt wird und der NH4-N Gehalt der flüssigen Phase auf unter 1000 ppm, bevorzugt auf unter 750 ppm, besonders bevorzugt auf unter 500 ppm reduziert wird. Das hat den Vorteil, dass mehr Ablauf aus der Biogasanlage in die Anmaischung zurückgeführt werden kann, ohne die Ethanolfermentation zu beeinträchtigen.

In einer weiteren Ausführungsform kann das erfindungsgemäße Verfahren so ausgestaltet sein, dass die flüssige, ammonium-arme Phase aus der Ammoniakstrippung einer Eindampfung unterzogen wird. In einer bevorzugten Ausführungsform werden der flüssigen, ammonium-armen Phase 0,7 t, bevorzugt 0,8 t, besonders bevorzugt 0,9 t Wasser pro Tonne flüssiger Phase als Eindampfkondensat entzogen. Dieser hohe Wasserentzug hat den Vorteil, dass ein nährstoffreiches Konzentrat, im Folgenden als Nährstoffkonzentrat bezeichnet, produziert wird, das als wertvoller Dünger verwendet werden kann. Des Weiteren wird weniger suspendierte Trockenmasse in die Ethanolanlage über den Ablauf der Biogasanlage zurückgeführt, was die Viskosität der Maische reduziert bzw. höhere Mengen Maismehl oder eine höhere Rückführung an Dick- und/oder Dünnschlempe zulässt. Eine solche Eindampfung ist zudem energetisch vorteilhaft gegenüber der typischen Dünnschlempeeindampfung zur Produktion von Prozesswasser, da eine effizientere Wärmerückgewinnung realisiert werden kann.

In einer weiteren Ausführungsform kann das erfindungsgemäße Verfahren so ausgestaltet sein, dass lignocellulosereiche Biomassen wie z.B. Stroh, Holz, Gräser, Bagasse, oder Sägespäne der Biogasanlage als Substrat zusätzlich zur Schlempe, zum Schlempefeststoff oder zu den Reststoffen aus der Ethanolanlage zugeführt werden. Dies hat im Vergleich zur Monovergärung dieser Stoffe den Vorteil, dass die Zugabe von Nährstoffen wie z.B. Natrium oder Stickstoff, die für das Wachstum der Mikroorganismen der Biogasanlage wichtig sind, reduziert wird, da diese bereits in den Reststoffen der Ethanolanlage vorliegen.

In einer weiteren Ausführungsform kann das erfindungsgemäße Verfahren so ausgestaltet sein, dass ein Teil des Biogases aus der Biogasanlage zur Erzeugung von Prozessenergie für die Ethanolanlage und/oder die Biogasanlage verwendet wird. Biogas kann z.B. in einem Blockheizkraftwerk in elektrische Prozessenergie umgewandelt werden. Biogas kann auch z.B. in einem Dampfkessel zur Dampferzeugung genutzt werden. In einer bevorzugten Ausführungsform kann das erfindungsgemäße Verfahren so ausgestaltet sein, dass der komplette Dampfbedarf der kombinierten Ethanol- und Biogasanlage durch das in der Biogasanlage erzeugte Biogas bereitgestellt wird. In einer bevorzugten Ausführungsform kann das erfindungsgemäße Verfahren so ausgestaltet sein, dass der komplette Energiebedarf der kombinierten Ethanol- und Biogasanlage durch das in der Biogasanlage erzeugte Biogas bereitgestellt wird, wobei übliche Anlagen nach dem Stand der Technik zur Erzeugung von Elektroenergie und Dampf aus Biogas zum Einsatz kommen können

In einer bevorzugten Ausführungsform ist die Kombination aus Bioethanol- und Biogasanlage so ausgestaltet, dass pro Tonne Maismehl maximal 0,5 m³, bevorzugt maximal 0,2 m³, besonders bevorzugt maximal 0,1 m³ Abwasser produziert werden, die aus der kombinierten Bioethanol- und Biogasanlage ausgeschleust werden.

In einer bevorzugten Ausführungsform ist das Verfahren so ausgestaltet, dass das Biogas einer Biogasreinigung zugeführt wird, in dem als Produkte mindestens CO₂ und Biomethan gewonnen werden. Das Biomethan kann z.B. verdichtet und in ein Erdgasnetz eingespeist oder als Fahrzeugtreibstoff eingesetzt werden.

### Kurzbeschreibung der Figuren

Die Erfindung wird nachfolgend anhand zweier Ausführungsbeispiele und dazugehörigen Zeichnungen näher erläutert.

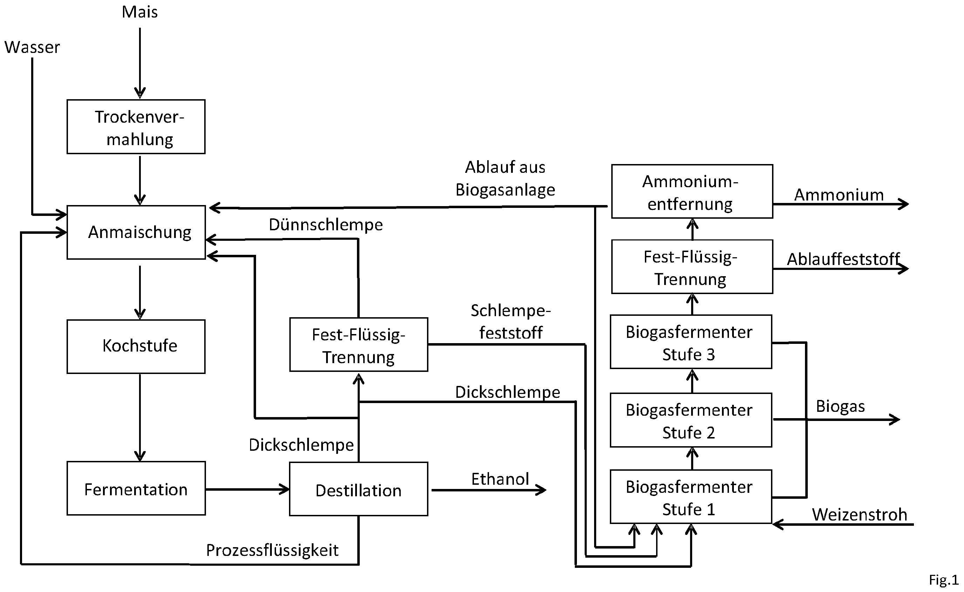

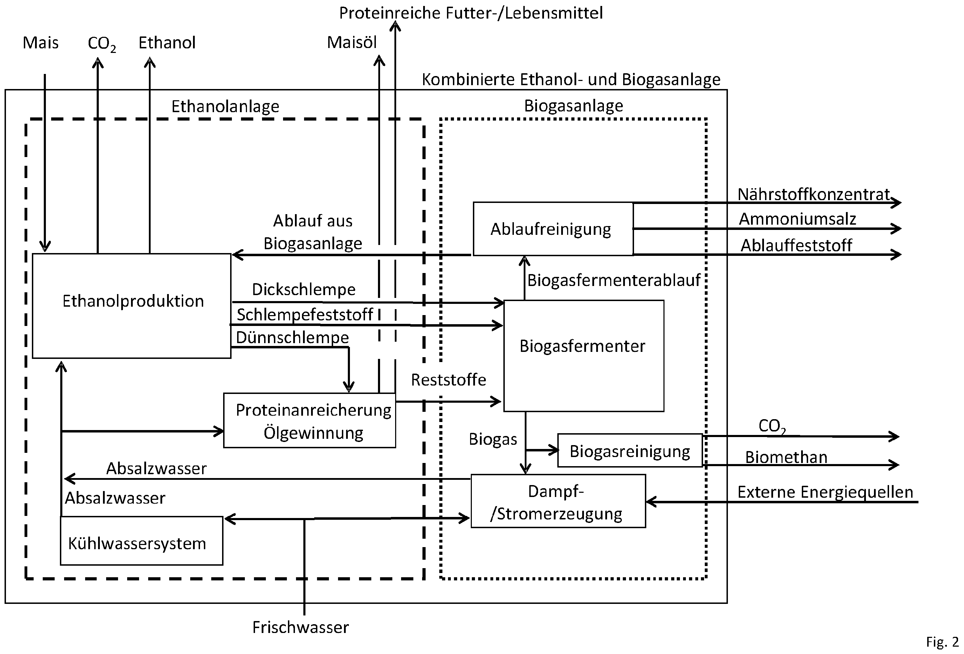

Fig. 1 und Fig.2 beschreiben den schematischen Prozessablauf von erfindungsgemäßen Ausführungsformen des Verfahrens.

### Ausführungsbeispiel 1

Figur 1 zeigt eine schematische Darstellung des Verfahrens ohne Futtermittel-, Lebensmittel- und Maisölherstellung. Tabelle 1 zeigt die Zusammensetzung des Maismehls dieses Ausführungsbeispiels. Tabelle 2 zeigt die Massenströme dieses Ausführungsbeispiels.

**Schritt 1:** Mais wird einer Trockenvermahlung zugeführt. Das Maismehl weist einen Massenanteil von 60 % an Partikeln kleiner 0,5 mm und einen TS-Gehalt von 85 % auf.

**Tabelle 1 Zusammensetzung Maismehl**

| | **Massenanteil an Originalsubstanz (OS)** | **Massenanteil an TS** |
|---|---|---|
| Wasser | 15,00 % | |
| Stärke | 60,90 % | 71,65 % |
| Zucker | 2,20 % | 2,59 % |
| Protein | 8,10 % | 9,53 % |
| Fett | 3,70 % | 4,35 % |
| Lignocellulose | 8,90 % | 10,47 % |
| Asche | 1,20 % | 1,41 % |
| Summe | 100,0 % | 100,0 % |

**Schritt 2:** 1,0 t/h Maismehl wird mit 0,4 t/h Ablauf aus der Biogasanlage, 0,8 t/h Dickschlempe, 1,6 t/h Dünnschlempe und 0,46 t/h Prozessflüssigkeit aus der Ethanolanlage und 0,12 t/h Wasser (z.B. Trinkwasser) angemaischt. Der Ablauf der Biogasanlage, Dickschlempe, Dünnschlempe und Prozessflüssigkeiten weisen TS-Gehalte von 4 %, 20,5 %, 15 % und ca. 0 % auf. Es werden somit pro Tonne Maismehl ca. 0,4 m³ Ablauf aus der Biogasanlage, 0,16 t TS Dickschlempe, und 0,4 t TS Schlempegemisch (Dickschlempe und Dünnschlempe) der Anmaischung zugeführt. Der Ablauf der Biogasanlage weist eine NH4-N Konzentration von 500 ppm auf. Damit werden 200 g NH4-N in Form von Ablauf aus der Biogasanlage pro Tonne Maismehl der Anmaischung zugeführt. Die Maische wird auf einen pH-Wert von 4,2 mit Schwefelsäure eingestellt und Enzyme zum Aufschluss der Stärke zugegeben (in Figur 1 und Massenbilanz Tabelle 2 vernachlässigt).

**Schritt 3a:** Die Maische aus Schritt 2 wird einer Kochstufe zugeführt und auf 60°C erhitzt.

**Schritt 3b:** Der Maische aus Schritt 3a wird auf unter 30°C gekühlt. Glucoamylase und Hefe werden zugeführt (in Figur 1 und Massenbilanz Tabelle 2 vernachlässigt) und Stärke in der Fermentation in Ethanol umgewandelt.

**Schritt 3c:** Die ethanolhaltige Maische wird einer Destillation bei 67°C zugeführt und 0,344 t/h Ethanol (entspricht 436 Liter pro Tonne Maismehl) aus der Maische entnommen. Der Ethanolstrom wird in weiteren Schritten vom Wasser befreit und der Wasserstrom als Prozessflüssigkeit in die Anmaischung gefahren. Die bei der Destillation entstehende ethanolarme Maische, die Dickschlempe, wird zu 0,8 t/h der Anmaischung (Schritt 2), zu 0,14 t/h der Biogasanlage (Schritt 5) und zu 2,3 t/h der Festflüssigtrennung (Schritt 4) zugeführt.

**Schritt 4:** 2,3 t/h Dickschlempe aus Schritt 3 werden in einer Dekantierzentrifuge einer Fest-Flüssigtrennung unterzogen. Dabei entstehen Dünnschlempe und Schlempefeststoff. Die Dünnschlempe wird zur Anmaischung (Schritt 2) zurückgeführt, der Schlempefeststoff wird der Biogasanlage (Schritt 5) zugeführt.

**Schritt 5a:** Dickschlempe aus Schritt 3 und Schlempefeststoff aus Schritt 4 und 0,14 t TS/h Weizenstroh werden den Biogasfermentern der ersten Stufe zugeführt. Der Ablauf der Biogasfermenter der ersten Stufe wird den Biogasfermentern der zweiten Stufe zugeführt. Der Ablauf der Biogasfermenter der zweiten Stufe wird den Biogasfermentern der dritten Stufe zugeführt. In allen drei Stufen entsteht Biogas. In der gesamten Biogasanlage werden 1,3 MW Biogas gebildet. Die durchschnittliche hydraulische Verweilzeit der gesamten Biogasanlage beträgt 70 Tage.

**Schritt 5b:** Der Ablauf des Biogasfermenters der dritten Stufe aus Schritt 5a wird in einer Dekantierzentrifuge einer Fest-Flüssig-Trennung zugeführt. Dabei entsteht Ablauffeststoff, der aus der Biogasanlage ausgeschleust wird.

**Schritt 5c:** Die flüssige Phase aus Schritt 5b wird einer Ammoniakstrippung (Ammoniumentfernung) zugeführt. Dabei wird der flüssigen Phase Ammoniak entzogen und der NH4-N Gehalt auf 500 ppm gesenkt. Das entzogene Ammonium wird in Form von Ammoniumsulfat aus der Biogasanlage ausgeschleust. 0,4 t/h Ablauf mit 500 ppm NH4-N wird der Anmaischung (Schritt 2) zugeführt. Ein Teil des Ablaufs wird in den Biogasfermenter in Schritt 5a zurückgefahren, um die NH4-N Konzentration im Biogasfermenter auf 6000-9000 ppm zu kontrollieren. Die Konzentrationen von organischen Säuren und aromatischen Verbindungen im Ablauf der Biogasanlage beträgt maximal je 150 ppm.

**Energiebilanz:** Mit dem Biogas, das in der Biogasanlage entsteht, lässt sich der gesamte Energiebedarf der kombinierten Bioethanol- und Biogasanlage decken. Das bedeutet für die produzierten Kraftstoffe, dass sich der CI-score deutlich im Vergleich zu einer typischen Bioethanolanlage verbessert. Bei einer typischen Bioethanolanlage vergrößern die Energieverbräuche den CI-score um ca. 20-25 gCO₂e/MJ.

**Wasserverbrauch:** Der Frischwasserbedarf der Anlage beträgt nur ca. 0,1 m3 pro Tonne Maismehl, was einer Reduktion an Frischwasser um eine Größenordnung im Vergleich zu einer typischen Bioethanolanlage entspricht.

**Tabelle 2 Ausführungsbeispiel 1 Massenströme**

| | **t / h** | **t TS / h** | **Kenngrößen** |
|---|---|---|---|
| **Trockenvermahlung (Schritt 1)** | | | |
| Körnermais, ein | ∼1 | ∼0,85 | |
| Maismehl zu Schritt 1 | 1,00 | 0,85 | |

| **Anmaischung (Schritt 2)** | | | |
|---|---|---|---|
| Maismehl aus Schritt 1 | 1,00 | 0,85 | |
| Ablauf Biogasanlage aus Schritt 5 | 0,40 | 0,02 | ∼ 0,4 m3 pro t Maismehl |
| Dickschlempe aus Schritt 3 | 0,80 | 0,16 | 0,16 t TS Dickschlempe pro t Maismehl |
| Dünnschlempe aus Schritt 4 | 1,60 | 0,24 | |
| Schlempegemisch (Dick- und Dünnschlempe, Rechengröße) | 2,40 | 0,40 | 0,40 t TS Schlempegemisch (Dick- und Dünnschlempe) pro t Maismehl |
| Prozessflüssiqkeit | 0,46 | ∼0 | |
| Wasser | 0,12 | ∼0 | |

### Ausführungsbeispiel 2

Figur 2 zeigt eine schematische Darstellung des Verfahrens mit Herstellung von hochwertigen, proteinreichen Futter- und Lebensmitteln und Maisöl.

**Schritt 1a:** Mais wird der Ethanolproduktion zugeführt. Diese enthält die Prozessschritte Trockenvermahlung, Anmaischung, Fermentation, Destillation und Fest-Flüssigtrennung eines Teils der Dickschlempe. Prozesswasser wird in der Form von Absalzwasser aus dem Kühlwassersystem und Absalzwasser aus dem System der Dampferzeugung zugeführt, sowie Ablauf aus der Biogasanlage zugeführt. Als Produkte werden Ethanol und Kohlenstoffdioxid und als Zwischenprodukte Dickschlempe, Schlempefeststoff und Dünnschlempe erzeugt.

**Schritt 1b:** Aus der Dünnschlempe aus Schritt 1a werden als Produkte hochwertige, proteinreiche Futter- und Lebensmittel, sowie Maisöl gewonnen. Proteinreich meint dabei, dass der Rohproteingehalt (Massenanteil Protein an TS) gegenüber der Dünnschlempe durch geeignete Verfahren gesteigert wurde. Als Zwischenprodukt entstehen Reststoffe, die einen geringeren Rohproteingehalt als die Dünnschlempe aufweisen.

**Schritt 2a:** Dickschlempe und Schlempefeststoff aus Schritt 1a sowie Reststoffe aus Schritt 1b werden den Biogasfermentern zugeführt. Als Zwischenprodukte entstehen Biogasfermenterablauf sowie Biogas.

**Schritt 2b:** Das Biogas aus Schritt 2a wird zur Erzeugung von Prozessenergie (Dampf, Strom) verwendet werden und/oder einer Biogasreinigung zugeführt, in der die Produkte Biomethan und Kohlenstoffdioxid entstehen.

**Schritt 2c:** Der Biogasfermenterablauf aus Schritt 2a wird einer Ablaufreinigung zugeführt. In einer Fest-Flüssigtrennung wird als Produkt ein Ablauffeststoff erzeugt, der z.B. als hochwertiger Dünger und Bodenverbesserer eingesetzt werden kann. Die flüssige Phase wird einer Ammoniakstrippung zugeführt, in der als Produkt Ammoniumsalz, ggf. als Ammoniumsalzlösung, gewonnen wird. Die ammoniumarme, flüssige Phase wird einer Eindampfung zugeführt, in der als Produkt ein Nährstoffkonzentrat gewonnen wird, das z.B. als Dünger eingesetzt werden kann. Als Zwischenprodukt entsteht aus den Kondensaten der Eindampfung ein Ablauf mit reduzierten Nährstoff-, Ammonium-, und Feststoffanteil im Vergleich zum Biogasfermenterablauf.

**Energiebilanz:** Abhängig von der produzierten Maisöl und Futter-/Lebensmittelmenge kann das in der Biogasanalage produzierte Biogas ausreichen, um den Gesamtenergiebedarf der kombinierten Ethanol- und Biogasanlage zu decken. Ggf. sind externe Energiequellen notwendig.

**Wasserverbrauch:** Der Frischwasserbedarf der Anlage beträgt nur ca. 0,1 m3 pro Tonne Maismehl, was einer Reduktion an Frischwasser um eine Größenordnung im Vergleich zu einer typischen Bioethanolanlage entspricht.

### Literaturverzeichnis

Jacques, K., Lyons, T., & Kelsall, D. (2003). The Alcohol Textbook 4th Edition. Nottingham, United Kingdom: Nottingham University Press.
Victor Guadalupe Medina, Marinka J. H. Almering, Antonius J. A. van Maris, Jack T. Pronk. Applied and Environmental Microbiology Dec 2009, 76 (1) 190-195; DOI: 10.1128/AEM.01772-09
Mueller, S., 2010b, Detailed Report: 2008 National Dry Mill Corn Ethanol Survey, Energy Resource Center, University of IL at Chicago, May.
Walker, G., Abbas, C., Ingledew, W., & Pilgrim, C. (2017). The Alcohol Textbook 6th Edition. Duluth, GA, USA: Lallemand Biofuels & Distilled Spirits.

## Patentansprüche

1. Verfahren zur Durchführung eines kombinierten Betriebs einer Bioethanolgewinnungsanlage und einer Biogasanlage, **dadurch gekennzeichnet, dass**
a) Maismehl aus einer Trockenvermahlung mindestens mit 0,1 t Trockensubstanz, TS, in Form von Dickschlempe und mindestens 0,1 m³ Ablauf der Biogasanlage pro Tonne Maismehl angemaischt wird,
b) die Maische aus Schritt a) einer Kochstufe mit Maischetemperaturen unterhalb der Verkleisterungstemperatur der Stärke im Maismehl, nachfolgend einer ethanolbildenden Fermentation und die fermentierte Maische nachfolgend einer Destillation zugeführt wird, und
c) Dickschlempe aus Schritt b) der Anmaischung in Schritt a) und der Biogasanlage zugeführt wird.

2. Verfahren nach Anspruch 1, **dadurch gekennzeichnet, dass** mindestens 0,2 t TS, bevorzugt mindestens 0,3 t TS, besonders bevorzugt mindestens 0,4 t TS in Form eines Gemischs aus Dünn- und Dickschlempe pro Tonne Maismehl in der Anmaischung in Schritt a) zugegeben werden.

3. Verfahren nach Anspruch 1, **dadurch gekennzeichnet, dass** mindestens 0,12 t TS, bevorzugt mindestens 0,14 t TS, besonders bevorzugt mindestens 0,16 t TS in Form von Dickschlempe pro Tonne Maismehl in die Anmaischung in Schritt a) zurückgeführt werden.

4. Verfahren nach Anspruch 1, **dadurch gekennzeichnet, dass** mindestens 0,1 t TS, bevorzugt mindestens 0,2 t TS, bevorzugt mindestens 0,25 t TS Dünnschlempe pro t Maische in die Anmaischung in Schritt a) zurückgeführt werden.

5. Verfahren nach Anspruch 1, **dadurch gekennzeichnet, dass** die Maische in der Kochstufe in Schritt b) auf maximal 70 °C, bevorzugt auf maximal 66 °C, besonders bevorzugt auf maximal 64 °C erwärmt wird.

6. Verfahren nach Anspruch 1, **dadurch gekennzeichnet, dass** das Maismehl in Schritt a) aus einer Trockenvermahlung mindestens einen Massenanteil von 60 %, bevorzugt mindestens von 65 %, besonders bevorzugt mindestens von 70 % an Partikeln mit einer Korngröße <0,5 mm aufweist.

7. Verfahren nach Anspruch 1, **dadurch gekennzeichnet, dass** der pH-Wert der Maische in Schritt a) auf kleiner 4,5, bevorzugt kleiner 4,2, besonders bevorzugt kleiner 3,8 eingestellt wird.

8. Verfahren nach Anspruch 1, **dadurch gekennzeichnet, dass** der Ammoniumstickstoffgehalt in den Biogasfermentern der Biogasanlage zwischen 6000-9000 ppm, bevorzugt zwischen 7000-9000 ppm, besonders bevorzugt zwischen 7500-9000 ppm gehalten wird.

9. Verfahren nach Anspruch 1, **dadurch gekennzeichnet, dass** die durchschnittliche hydraulische Verweilzeit der Biogasanlage mindestens 30 Tage, bevorzugt mindestens 50 Tage, besonders bevorzugt mindestens 70 Tage beträgt.

10. Verfahren nach Anspruch 1, **dadurch gekennzeichnet, dass** der Anteil an Ablauf aus der Biogasanlage pro Tonne Maismehl in Schritt a) mindestens 0,2 m³, bevorzugt mindestens 0,4 m³, besonders bevorzugt mindestens 0,8 m³ beträgt.

11. Verfahren nach Anspruch 1, **dadurch gekennzeichnet, dass** pro Tonne Maismehl in der Destillation in Schritt b) mindestens 400 Liter Ethanol, bevorzugt mindestens 425 Liter Ethanol, besonders bevorzugt mindestens 435 Liter Ethanol abgetrennt werden.

12. Verfahren nach Anspruch 1, **dadurch gekennzeichnet, dass** in der Anmaischung in Schritt a) mindestens 100 g, bevorzugt mindestens 200 g, besonders bevorzugt mindestens 400 g Ammoniumstickstoff pro Tonne Maismehl über den Ablauf der Biogasanlage zurückgeführt werden.

13. Verfahren nach Anspruch 1, **dadurch gekennzeichnet, dass** in der Anmaischung in Schritt a) maximal 1000 g, bevorzugt maximal 800 g, besonders bevorzugt maximal 600 g Ammoniumstickstoff pro Tonne Maismehl über den Ablauf der Biogasanlage zurückgeführt werden.

14. Verfahren nach Anspruch 1, **dadurch gekennzeichnet, dass** pro Tonne Maismehl weniger als 1,2 m³, bevorzugt weniger als 0,8 m³, besonders bevorzugt weniger als 0,2 m³ Frischwasser eingesetzt werden.

15. Verfahren nach Anspruch 1, **dadurch gekennzeichnet, dass** pro Tonne Maismehl mindestens 15 kg, bevorzugt mindestens 18 kg, besonders bevorzugt mindestens 20 kg Glycerin über die Dick- und Dünnschlempe in die Anmaischung zurückgefahren wird.

16. Verfahren nach Anspruch 1, **dadurch gekennzeichnet, dass** ein Massenanteil von Cellulose an der TS der Dickschlempe mindestens 5 % beträgt und über den Ablauf aus der Biogasanlage Cellulasen und cellulasebildenden Mikroorganismen aus der Biogasanlage der Anmaischung zugeführt werden, wobei der Cellulosegehalt als acid detergent fiber'-Messwert, ADF, abzüglich einem acid detergent lignin'-Messwert, ADL, einer Dickschlempeprobe berechnet wird.

## Claims

1. A method for carrying out a combined operation of a bioethanol production unit and a biogas unit, **characterized in that**:
a) corn meal from a dry milling step is mashed with at least 0.1 t of dry matter, i.e., TS, in the form of whole stillage and at least 0.1 m³ of outflow from the biogas unit per tonne of corn meal,
b) the mash from step a) is fed to a cooking stage with mash temperatures below the gelatinization temperature of the starch in the corn meal, followed by an ethanol-forming fermentation step and then feeding the fermented mash to a distillation step, and
c) the whole stillage from step b) is fed to the mashing step in step a) and to the biogas unit.

2. The method according to claim 1, **characterized in that** at least 0.2 t TS, preferably at least 0.3 t TS, particularly preferably at least 0.4 t TS per tonne of corn meal is added to the mashing step in step a) in the form of a mixture of thin stillage and whole stillage.

3. The method according to claim 1, **characterized in that** at least 0.12 t TS, preferably at least 0.14 t TS, particularly preferably at least 0.16 t TS per tonne of corn meal is recycled to the mashing step in step a) in the form of whole stillage.

4. The method according to claim 1, **characterized in that** at least 0.1 t TS, preferably at least 0.2 t TS, preferably at least 0.25 t TS of thin stillage per t of mash is recycled to the mashing step in step a).

5. The method according to claim 1, **characterized in that** in the cooking stage in step b), the mash is heated to a maximum of 70°C, preferably to a maximum of 66°C, particularly preferably to a maximum of 64°C.

6. The method according to claim 1, **characterized in that** the corn meal in step a) from a dry milling step has a mass fraction of at least 60%, preferably at least 65%, particularly preferably at least 70% of particles with a particle size of < 0.5 mm.

7. The method according to claim 1, **characterized in that** the pH of the mash in step a) is adjusted to less than 4.5, preferably to less than 4.2, particularly preferably to less than 3.8.

8. The method according to claim 1, **characterized in that** the ammonium nitrogen content in the biogas fermenters of the biogas unit is kept at between 6000-9000 ppm, preferably between 7000-9000 ppm, particularly preferably between 7500-9000 ppm.

9. The method according to claim 1, **characterized in that** the mean hydraulic residence time for the biogas unit is at least 30 days, preferably at least 50 days, particularly preferably at least 70 days.

10. The method according to claim 1, **characterized in that** the proportion of outflow from the biogas unit per tonne of corn meal in step a) is at least 0.2 m³, preferably at least 0.4 m³, particularly preferably at least 0.8 m³.

11. The method according to claim 1, **characterized in that** in the distillation step in step b), at least 400 liters of ethanol, preferably at least 425 liters of ethanol, particularly preferably at least 435 liters of ethanol are separated per tonne of corn meal.

12. The method according to claim 1, **characterized in that** in the mashing step in step a), at least 100 g, preferably at least 200 g, particularly preferably at least 400 g of ammonium nitrogen per tonne of corn meal is recycled via the outflow from the biogas unit.

13. The method according to claim 1, **characterized in that** in the mashing step in step a), a maximum of 1000 g, preferably a maximum of 800 g, particularly preferably a maximum of 600 g of ammonium nitrogen per tonne of corn is recycled via the outflow from the biogas unit.

14. The method according to claim 1, **characterized in that** less than 1.2 m³, preferably less than 0.8 m³, particularly preferably less than 0.2 m³ of fresh water is used per tonne of corn meal.

15. The method according to claim 1, **characterized in that** at least 15 kg, preferably at least 18 kg, particularly preferably at least 20 kg of glycerine per tonne of corn meal is recycled to the mashing step via the whole stillage and thin stillage.

16. The method according to claim 1, **characterized in that** a mass fraction of cellulose in the TS of the whole stillage is at least 5% and that cellulases and cellulase-forming microorganisms from the biogas unit are fed to the mashing step via the outflow from the biogas unit, wherein the cellulose content is calculated as the measured acid detergent fiber, i.e., ADF, minus the measured acid detergent lignin, i.e., ADL, of a whole stillage sample.

## Revendications

1. Procédé pour réaliser un fonctionnement combiné d'une installation de production de bioéthanol et d'une installation de biogaz, **caractérisé en ce que**
a) la farine de maïs issue d'une mouture sèche est mélangée à au moins 0,1 tonne de matière sèche, MS, sous forme de drêches épaisses et à au moins 0,1 m³ d'effluent de l'installation de production de biogaz par tonne de farine de maïs,
b) le moût de l'étape a) est soumis à une étape de cuisson avec des températures de moût inférieures à la température de gélatinisation de l'amidon dans la farine de maïs, puis à une fermentation formant de l'éthanol, et le moût fermenté est ensuite soumis à une distillation, et
c) les drêches épaisses de l'étape b) sont envoyées à l'empâtage de l'étape a) et à l'installation de biogaz.

2. Procédé selon la revendication 1, **caractérisé en ce qu'**au moins 0,2 t de MS, de préférence au moins 0,3 t de MS, de manière particulièrement préférée au moins 0,4 t de MS sous la forme d'un mélange de drêches fines et de drêches épaisses par tonne de farine de maïs sont ajoutées dans le mélange d'empâtage à l'étape a).

3. Procédé selon la revendication 1, **caractérisé en ce qu'**au moins 0,12 t de MS, de préférence au moins 0,14 t de MS, de manière particulièrement préférée au moins 0,16 t de MS sous forme de drêches épaisses par tonne de farine de maïs est recyclée dans l'empâtage à l'étape a).

4. Procédé selon la revendication 1, **caractérisé en ce qu'**au moins 0,1 t de MS, de préférence au moins 0,2 t de MS, de préférence au moins 0,25 t de MS de drêches fines par tonne de moût sont recyclées l'empâtage à l'étape a).

5. Procédé selon la revendication 1, **caractérisé en ce que** le moût est chauffé dans l'étape de cuisson de l'étape b) à 70°C maximum, de préférence à 66°C maximum, de manière particulièrement préférée à 64°C maximum.

6. Procédé selon la revendication 1, **caractérisé en ce que** la farine de maïs à l'étape a) provenant d'une mouture sèche présente au moins une proportion massique de 60 %, de préférence d'au moins 65 %, de manière particulièrement préférée d'au moins 70 % de particules ayant une taille de grain <0,5 mm.

7. Procédé selon la revendication 1, **caractérisé en ce que** le pH du moût est ajusté à l'étape a) à une valeur inférieure à 4,5, de préférence inférieure à 4,2, de manière particulièrement préférée inférieure à 3,8.

8. Procédé selon la revendication 1, **caractérisé en ce que** la teneur en azote ammoniacal dans les fermenteurs de biogaz de l'installation de biogaz est maintenue entre 6000-9000 ppm, de préférence entre 7000-9000 ppm, de manière particulièrement préférée entre 7500-9000 ppm.

9. Procédé selon la revendication 1, **caractérisé en ce que** le temps de séjour hydraulique moyen de l'installation de biogaz est d'au moins 30 jours, de préférence d'au moins 50 jours, de manière particulièrement préférée d'au moins 70 jours.

10. Procédé selon la revendication 1, **caractérisé en ce que** la proportion d'effluent provenant de l'installation de biogaz par tonne de farine de maïs dans l'étape a) est d'au moins 0,2 m³, de préférence d'au moins 0,4 m³, de manière particulièrement préférée d'au moins 0,8 m³.

11. Procédé selon la revendication 1, **caractérisé en ce que**, par tonne de farine de maïs, au moins 400 litres d'éthanol, de préférence au moins 425 litres d'éthanol, de manière particulièrement préférée au moins 435 litres d'éthanol sont séparés dans la distillation de l'étape b).

12. Procédé selon la revendication 1, **caractérisé en ce que**, dans l'empâtage à l'étape a), au moins 100 g, de préférence au moins 200 g, de manière particulièrement préférée au moins 400 g d'azote ammoniacal par tonne de farine de maïs sont recyclés via la sortie de l'installation de biogaz.

13. Procédé selon la revendication 1, **caractérisé en ce que** dans le l'empâtage à l'étape a), un maximum de 1000 g, de préférence un maximum de 800 g, de manière particulièrement préférée un maximum de 600 g d'azote ammoniacal par tonne de farine de maïs est recyclé via l'effluent de l'installation de biogaz.

14. Procédé selon la revendication 1, **caractérisé en ce que** moins de 1,2 m³, de préférence moins de 0,8 m³, de manière particulièrement préférée moins de 0,2 m³ d'eau fraîche sont utilisés par tonne de farine de maïs.

15. Procédé selon la revendication 1, **caractérisé en ce que**, par tonne de farine de maïs, au moins 15 kg, de préférence au moins 18 kg, de manière particulièrement préférée au moins 20 kg de glycérol sont recyclés dans le mélange de maïs par l'intermédiaire des drêches épaisses et des drêches fines.

16. Procédé selon la revendication 1, **caractérisé en ce qu'**une proportion en masse de cellulose par rapport à la MS des drêches épaisses est d'au moins 5 % et **en ce que** des cellulases et des micro-organismes formant des cellulases provenant de l'installation de biogaz sont ajoutés à l'empâtage par l'intermédiaire de l'effluent de l'installation de biogaz, la teneur en cellulose étant calculée en tant que valeur de mesure 'acid detergent fiber', ADF, moins une valeur de mesure 'acid detergent lignin', ADL, d'un échantillon de drêches épaisses.
